(19) Europäisches Patentamt
European Patent Office
Office européen des brevets

(11) Publication number: **0 132 545 B1**

(12) # EUROPEAN PATENT SPECIFICATION

(45) Date of publication of patent specification: **27.02.91**

(51) Int. Cl.$^5$: **C07C 17/00**, C07C 21/04, C07C 21/067

(21) Application number: **84106232.6**

(22) Date of filing: **30.05.84**

The file contains technical information submitted after the application was filed and not included in this specification

(54) **Isomerization of allylic halides with organic amines.**

(30) Priority: **20.07.83 US 515565**

(43) Date of publication of application:
**13.02.85 Bulletin 85/07**

(45) Publication of the grant of the patent:
**27.02.91 Bulletin 91/09**

(84) Designated Contracting States:
**BE CH DE FR GB LI NL**

(56) References cited:
**FR-A- 2 020 235**
**GB-A- 798 889**
**US-A- 3 819 730**

(73) Proprietor: **Union Camp Corporation**
**1600 Valley Road**
**Wayne New Jersey 07470 - 2066(US)**

(72) Inventor: **Mitchell, Peter W.D.**
**106 Lancaster Road**
**Freehold New Jersey 07728(US)**
Inventor: **McElligott, Lois T.**
**1158 Wheatsheaf Lane**
**Abington Pennsylvania 19001(US)**

(74) Representative: **von Füner, Alexander, Dr. et al**
**Patentanwälte v. Füner, Ebbinghaus, Finck**
**Mariahilfplatz 2 & 3**
**D-8000 München 90(DE)**

**EP 0 132 545 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The invention relates to the isomerization of allylic halides.

Representative of the prior art is the description given in the UK-A-798,889. In FR-A-2,020,235 there is described a process for the isomerization of 3,4-dichlorobutene-1 to 1,4-dichlorobutene-2, wherein dichlorobutene is reacted under anhydrous conditions with a mixture of a chlorohydrate of an organic amine and at least a substance from the group of metals and metal salts as catalyst. Preferred as a metal is copper or certain copper salts as well as salts of iron, aluminium, titanium, zinc and bismuth. A preferred chlorohydrate of the organic amino compounds are lower alkyl, amine, aniline, α-naphthylamine, β-naphthylamine, diphenylamine, p-phenylene diamine, m-phenylene diamine, N,N-dimethylaniline and benzidine. In spite of the availability of several known methods of isomerizing allylic halides, there has remained a need for economical processes, saving of energy. The method of the present invention is such an improvement over the prior art, requiring low temperatures and short isomerization times. The method of the invention is particularly valuable for the isomerization of heat sensitive compounds such as the linalyl, geranyl and neryl halides. Improved yields result.

The invention comprises in a method for isomerizing an allylic halide to its allylic isomer which comprises isome rizing the allylic halide in the presence of an organic amine and a catalytic portion of a copper catalyst, the improvement which comprises carrying out the isomerization in the presence of a mixture of tri(alkyl)amines where the alkyl portion comprises a chain of from 6 to 10 carbons.

The term "halide" as used herein is embracive of chloride, bromide and iodide.

It is preferred that the process is carried out at a temperature of from 0 °C to 60 °C.

According to a preferred embodiment cuprous chloride is used as copper catalyst.

The copper catalyst should especially be used in a weight range of 0.01 to 2.0% of allylic halide wherein the molar ratio of organic amine to copper catalyst is 0.1 to 5.1.

It is furthermore preferred to carry out the isomerization for a period of 0.1 to 10.0 hours.

The mixture of tri(alkyl)amines where the alkyl portion comprises a chain of from six to ten carbons as the organic amine may be selected from the group consisting of trihexylamine, trioctylamine, tridecylamine, di(hydrogenated tallow) methyl amine, distearyl methyl amine and octadecylamine.

The isomerization is especially carried out on linalyl chloride or 3-chloro-3-methyl-1-butene.

The method of the invention may be employed to isomerize any allylic halide to obtain its allylic isomer. In other words, a tertiary allylic halide may be isomerized to the corresponding secondary or primary allylic halide and the secondary allylic halide may be isomerized to the corresponding primary allylic halide. Representative of an advantageous isomerization according to the method of the invention is the isomerization of linalyl chloride to the geranyl and neryl chlorides according to the schematic formulae:-

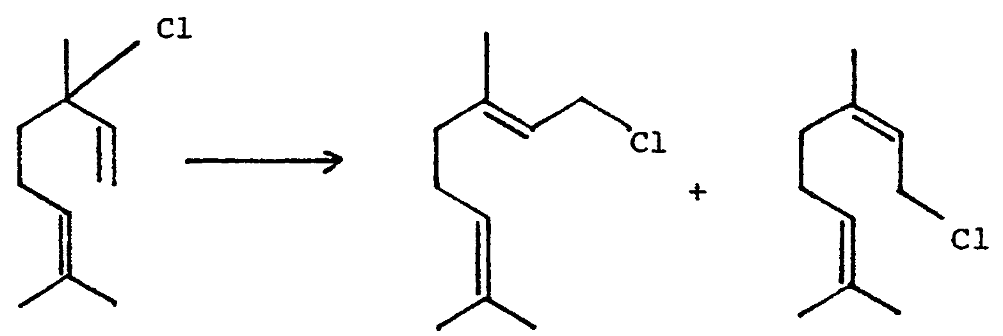

linalyl chloride — geranyl chloride — neryl chloride

The method of the invention is not limited to the isomerization of linalyl chloride to geranyl and neryl chlorides but may be used to isomerize any allylic halide to its allylic isomer. Other representative allylic halides which may be advantageously isomerized include 3,4-dichloro-1-butene, 3-chloro-3-methyl-1-butene and like allylic halides.

Although the method of the invention is not dependent upon the presence of hydrogen halide, it is preferred to employ a catalytic proportion of a hydrogen halide. Catalytic proportions are generally within the range of from about 0.01 to 5 percent by weight of the starting halides, preferably 0.1 to 1.0 percent.

The copper catalysts employed may be any copper compound having a valency of 2 or less, including metallic copper. Any copper compound convertible to the halide such as the bromide, iodide or chloride

under conditions of the reaction may also be used. Representative of copper catalysts advantageously employed are the chloride, bromide, carbonate, oxide, acetate, formate, sulfate and like derivative cupric and cuprous compounds. Preferred as the copper catalyst in the improved process of the invention is cuprous chloride. Catalytic proportions of the copper catalyst are generally within the weight range of from about 0.01 to 2 percent of the allylic halide starting compound, preferably about 0.5 percent.

It will be appreciated that under specific conditions of operating the process of the invention, certain of the above described compounds of the formula (I) given above have advantages over other compounds of the same general formula. Selection of a particular compound (I) for use under specific process conditions for optimum yields may be made by trial and error techniques. We have observed however that there are advantages associated with a mixture of trialkylamines where the alkyl portion consists of a chain of from 6 to 10 carbon atoms. For example, Adogen 364 (Sherex Chemical Co.) .

The organic amine is used in a proportion to isomerize at least some of the allylic halide according to the method of the invention. Such a proportion is generally within the range of from about 0.01 to 10 percent by weight of the halide charge, preferably 0.2 to 2.5 percent. Optimum proportions will depend to some extent upon the salt selected and may be determined by trial and error technique. Generally the preferred molar ratio of compound (I) to copper catalyst is from 0.01 to 5.0.

The method of the invention may be carried out by admixing the starting allylic halide with the hydrogen halide, copper catalyst and amine compound of the formula (I) in a suitable reaction vessel for a sufficient period of time to effect the desired isomerization. The controlling reaction rate in the method of the invention is the isomerization of the allylic halide to the desired allylic isomer. This is controlled by residence time in the reaction zone. We have found that in isomerization of linalyl chloride the preferred minimum total residence time is within the range of from 0.1 to 10 hours and most preferably 5 to 8 hours under preferred operating temperatures.

Although the method of the invention may be carried out under a wide range of operating temperatures, i.e. within the range of from about 0 ° C. to 60 ° C., it is preferred to do so at a temperature of from 0 ° C to 30 ° C. and most preferably 0 ° C. to 20 ° C.

The method of the invention is not dependent upon pressure, and may be carried out at atmospheric, sub-atmospheric or super-atmospheric pressures.

Progress of the isomerization may be monitored by conventional analytical techniques. When it has been determined that isomerization occurred to a maximum desired point, the product mixture may be passed from the reaction apparatus. The desired allylic halide isomer may then be separated from the reaction mixture employing conventional and known techniques including washing, decantation, distillation and like techniques.

The following examples describe the manner and process of making and using the invention and set forth the best mode contemplated by the invention of carrying out the invention but are not to be construed as limiting All parts given are by weight unless otherwise indicated.

EXAMPLES 1 and 2

To 6.0 g of isoprene hydrochlorides consisting of 65 parts 3-chloro-3-methyl-1-butene (abbreviated 3,3,1-CMB) and 35 parts 1-chloro-3-methyl-2-butene (abbreviated 1,3,2-CMB) was added 0.03 g cuprous chloride, 0.02 g hydrogen chloride gas and an amine compound as described in the table below. Each mixture was stirred at 10 ° C for 6 hours. Samples were withdrawn after 2 hours and 6 hours, neutralized with aqueous sodium hydroxide, and analyzed by nuclear magnetic resonance spectroscopy. The results obtained are shown in the table below.

## TABLE

| Example No. | Amine and Amount Used | Production Composition After 6 hours (%) | |
|---|---|---|---|
| | | 3,3,1-CMB | 1,3,2-CMB |
| 1 | Adogen 364*®, 0.13g | 13 | 87 |
| 2 | Di(hydrogenated tallow) methyl amine, 0.17g | 42 | 58 |

* Sherex Chemical Co., a mixture of Tri ($C_6$-$C_{12}$) amines wherein the carbon chains comprise: 1% $C_6$; 59% $C_8$; 39% $C_{10}$; and 1% $C_{12}$.

## Claims

1. In a method for isomerizing an allylic halide to its allylic isomer which comprises isomerizing the allylic halide in the presence of an organic amine and of a catalytic portion of a copper catalyst, the improvement which comprises; carrying out the isomerization in the presence of a mixture of tri(alkyl)-amines where the alkyl portion comprises a chain of from 6 to 10 carbons.

2. The process of claim 1 wherein the temperature is 0 ° C to 60 ° C.

3. The process of claim 1 or 2 wherein the copper catalyst is cuprous chloride.

4. The process of one of the claims 1 to 3 wherein the catalytic proportion of copper catalyst is within the weight range of 0.01 to 2.0 % of allylic halide.

5. The process of one of the claims 1 to 4 wherein the molar ratio of organic amine to copper catalyst is 0.1 to 5.0.

6. The process of one of the claims 1 to 5 wherein the isomerization is carried out for a period of 0.1 to 10.0 hours.

7. The process of one of the claims 1 to 6 wherein the organic amine is selected from a group consisting of trihexylamine, trioctylamine and tridecylamine, di(hydrogenated tallow) methyl amine, distearyl methyl amine and octadecylamine.

8. The process of claims 1 to 7 wherein the allylic halide is linalyl chloride.

9. The process of claim 1 wherein the allylic halide is 3-chloro-3-methyl-1-butene.

## Revendications

1. Dans un procédé d'isomérisation d'un halogénure allylique en son isomère allylique qui comprend l'isomérisation de l'halogénure allylique en présence d'une amine organique et d'une portion catalytique d'un catalyseur à base de cuivre, le perfectionnement qui comprend la réalisation de l'isomérisation en présence d'un mélange de tri(alkyl)amines dans lesquelles la partie alkyle comprend une chaîne de 6 à 10 atomes de carbone.

2. Procédé selon la revendication 1 dans lequel la température est comprise entre 0 ° C et 60 ° C.

3. Procédé selon la revendication 1 ou 2 dans lequel le catalyseur à base de cuivre est le chlorure cuivreux.

4. Procédé selon l'une des revendications 1 à 3 dans lequel la proportion catalytique du catalyseur à base de cuivre est comprise dans une gamme de poids de 0,01 à 2,0% de l'halogénure allylique.

5. Procédé selon l'une des revendications 1 à 4 dans lequel le rapport molaire entre amine organique et catalyseur à base de cuivre est de 0,1 à 5,0.

6. Procédé selon l'une des revendications 1 à 5 dans lequel l'isomérisation est réalisée pendant une période de temps comprise entre 0,1 et 10 heures.

7. Procédé selon l'une des revendications 1 à 6 dans lequel l'amine organique est choisie dans un groupe constitué par la trihexylamine, la trioctylamine, la tridécylamine, la di(suif hydrogéné)méthyl amine, la distéaryl méthyl amine et l'octadécylamine.

8. Procédé selon les revendications 1 à 7 dans lequel l'halogénure allylique est le chlorure de linalyle.

9. Procédé selon la revendication 1 dans lequel l'halogénure allylique est le 3-chloro-3-méthyl-1-butène.

## Ansprüche

1. In einem Verfahren zur Isomerisierung eines Allylhalogenids zu dessen Allylisomeren, wobei das Allylhalogenid in Gegenwart eines organischen Amins und eines katalytischen Anteils an Kupferkatalysator isomerisiert wird, besteht die Verbesserung in der Durchführung der Isomerisierung in Gegenwart eines Gemisches von Tri(alkyl)aminen, worin der Alkylanteil eine Kette von 6 bis 10 Kohlenstoffatomen aufweist.

2. Verfahren nach Anspruch 1, wobei die Temperatur 0 bis 60 ° C beträgt.

3. Verfahren nach Anspruch 1 oder 2, wobei der Kupferkatalysator Kupfer(I)-chlorid ist.

4. Verfahren nach einem der Ansprüche 1 bis 3, wobei der katalytische Anteil an Kupferkatalysator zwischen 0,01 und 2,0 Gewichts-%, bezogen auf das Allylhalogenid, beträgt.

5. Verfahren nach einem der Ansprüche 1 bis 4, wobei das Molverhältnis von organischem Amin zu Kupferkatalysator 0,1 bis 5,0 beträgt.

6. Verfahren nach einem der Ansprüche 1 bis 5, wobei die Isomerisierung 0,1 bis 10 Stunden lang durchgeführt wird.

7. Verfahren nach einem der Ansprüche 1 bis 6, wobei das organische Amin ausgewählt ist aus der Gruppe, bestehend aus Trihexylamin, Trioctylamin und Tridecylamin, Di(hydriertes Talg)-methylamin, Distearylmethylamin und Octadecylamin.

8. Verfahren nach einem der Ansprüche 1 bis 7, wobei das Allylhalogenid Linalylchlorid ist.

9. Verfahren nach Anspruch 1, wobei das Allylhalogenid 3-Chlor-3-methyl-1-buten ist.